(19) Europäisches Patentamt
European Patent Office
Office européen des brevets

(11) Veröffentlichungsnummer: **0 053 327**
**B1**

(12) # EUROPÄISCHE PATENTSCHRIFT

(45) Veröffentlichungstag der Patentschrift: **30.04.86**

(51) Int. Cl.⁴: **C 07 D 251/70**, C 08 G 18/80, C 08 G 18/38

(21) Anmeldenummer: **81109757.5**

(22) Anmeldetag: **19.11.81**

(54) **Verfahren zur Herstellung von s-Triazineinheiten und Isocyanatgruppen oder gegenüber Isocyanatgruppen reaktionsfähige Gruppen aufweisenden Verbindungen und ihre Verwendung als einbaufähige Füllstoffe bei der Herstellung von Polyurethanen.**

(30) Priorität: **02.12.80 DE 3045470**

(43) Veröffentlichungstag der Anmeldung: **09.06.82 Patentblatt 82/23**

(45) Bekanntmachung des Hinweises auf die Patenterteilung: **30.04.86 Patentblatt 86/18**

(84) Benannte Vertragsstaaten: **BE DE FR GB IT**

(56) Entgegenhaltungen: **EP - A - 0 000 893**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Grögler, Gerhard, Dr., von-Diergardt-Strasse 46, D-5090 Leverkusen (DE)**
Erfinder: **Rasshofer, Werner, Dr., Wolfskaul 10, D-5000 Köln 80 (DE)**
Erfinder: **Kopp, Richard, Dr., Wolfskaul 12, D-5000 Köln 80 (DE)**

**EP 0 053 327 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein Verfahren zur Herstellung von unzersetzt schmelzbaren, s-Triazineinheiten und Isocyanatgruppen oder gegenüber Isocyanatgruppen reaktionsfähige Gruppen aufweisenden Verbindungen durch Umsetzung von s-Triazineinheiten aufweisenden Triisocyanaten mit Verbindungen mit gegenüber Isocyanatgruppen reaktionsfähigen Gruppen, sowie die Verwendung der erfindungsgemäßen Verfahrensprodukte als einbaufähige Füllstoffe bei der Herstellung von Polyurethankunststoffen nach dem Isocyanat-Polyadditionsverfahren.

In der europäischen Patentschrift 893 werden s-Triazineinheiten aufweisende Polyisocyanate beschrieben, die durch Umsetzung von Melamin mit überschüssigen Mengen an aromatischen Diisocyanaten mit Isocyanatgruppen einer unterschiedlichen Reaktivität erhalten werden. Diese in der Vorveröffentlichung beschriebenen Verbindungen stellen in organischen Medien schwer- bis unlösliche Festkörper dar, die sehr hochschmelzend sind und sich in der Schmelze zersetzen. Wegen ihres hohen Schmelzpunktes und ihrer schlechten Löslich- bzw. Verträglichkeit sind der Verwendbarkeit der genannten Polyisocyanate als reaktive Füllstoffe bei der Herstellung von Polyurethanen nach dem Isocyanat-Polyadditionsverfahren enge Grenzen gesetzt.

Es war daher die Aufgabe der vorliegenden Erfindung, ausgehend von den bekannten s-Triazineinheiten aufweisenden Polyisocyanaten einbaufähige Füllstoffe zur Verfügung zu stellen, die nicht mit den genannten Nachteilen behaftet sind, d.h. die unzersetzt schmelzbar sind und/oder die eine verbesserte Verträglichkeit mit den zur Herstellung von Polyurethankunststoffen eingesetzten Ausgangsmaterialien aufweisen.

Diese Aufgabe konnte durch das nachstehend näher beschriebene erfindungsgemäße Verfahren gelöst werden, bei welchem s-Triazineinheiten aufweisende Polyisocyanate der in der europäischen Patentschrift 893 beschriebenen Art mit bestimmten difunktionellen Verbindungen mit gegenüber Isocyanatgruppen reaktionsfähigen Gruppen, wie nach stehend näher erläutert, modifiziert werden. Es war nicht vorhersehbar, daß durch diese erfindungsgemäße Modifizierung der genannten Polyisocyanate einbaufähige Füllstoffe bzw. Zusatzmittel zugänglich sein würden, die im Unterschied zu den Polyisocyanaten gemäß Vorveröffentlichung insbesondere eine verbesserte Verträglichkeit mit den zur Herstellung von Polyurethankunststoffen eingesetzten Ausgangsmaterialien aufweisen und vermutlich aus diesem Grunde weit besser dazu geeignet sind, die mechanischen Eigenschaften der letztendlich erhaltenen Kunststoffe zu verbessern.

Gegenstand der Erfindung ist somit ein Verfahren zur Herstellung von unzersetzt schmelzbaren, als einbaufähige Füllstoffe für Polyurethankunststoffe geeigneten, s-Triazineinheiten und Isocyanatgruppen oder gegenüber Isocyanatgruppen reaktionsfähige Gruppen aufweisenden Verbindungen, mit einem maximalen Molekulargewicht von 2000, dadurch gekennzeichnet, daß man Triisocyanate der Formel.

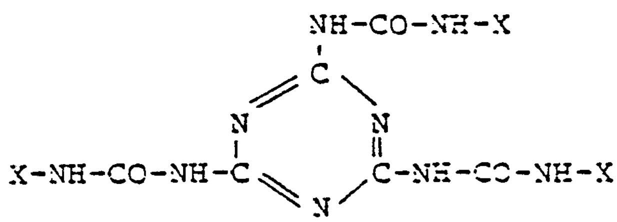

in welcher

X für einen Rest steht, wie er durch Entfernung der höher-reaktiven Isocyanatgroupe aus einem aromatischen Diisocyanat mit Isocyanatgruppen unterschiedlicher Reaktivität im Sinne der Isocyanat-Additionsreaktion erhalten wird,

mit mindestens difunktionellen Verbindungen eines maximalen Molekulargewichts von 300 mit gegenüber Isocyanatgruppen reaktionsfähigen Gruppen unter Einhaltung eines Äquivalentverhältnisses zwischen Isocyanatgruppen und gegenüber Isocyanatgruppen reaktionsfähigen Gruppen von mindestens 1,5:1 oder unter Einhaltung eines Äquivalentverhältnisses zwischen gegenüber Isocyanatgruppen reaktionsfähigen Gruppen und Isocyanatgruppen von mindestens 1,5:1 zur Reaktion bringt.

Gegenstand der Erfindung ist auch die Verwendung der erfindungsgemäßen Verfahrensprodukte als einbaufähige Füllstoffe bei der Herstellung von Polyurethankunststoffen nach dem Isocyanat-Polyadditionsverfahren.

Beim erfindungsgemäßen Verfahren werden s-Triazineinheitenn aufweisende Polyisocyanate der o.g. Formel eingesetzt, wobei X die o.g. Bedeutung hat. Vorzugsweise kommen solche Polyisocyanate der genannten Formel zum Einsatz, für welche X für einen 3-Isocyanato-4-methyl-phenyl- oder einen 4-(2-Isocyanatobenzyl)-phenylrest steht. Die Herstellung derartiger Polyisocyanate ist beispielsweise in EP-PS 893 beschrieben.

Für das erfindungsgemäße Verfahren geeignete s-Triazineinheiten aufweisende Polyisocyanate sind demzufolge insbesondere N,N',N''-Tris-(3-isocyanato-4-methyl-phenyl-aminocarbonyl)-melamin oder N,N',N''-Tris-[4-(2-isocya-natobenzyl)-phenyl-aminocarbonyl] -melamin. Es können jedoch beim erfindungsgemäßen Verfahren auch beliebige andere, obiger Definition entsprechende s-Triazineinheiten aufweisende aromatische Polyisocyanate eingesetzt werden.

Reaktionspartner für die genannten s-Triazineinheiten aufweisenden Polyisocyanate sind beliebige, im Sinne der Isocyanat-Additionsreaktion mindestens difunktionelle Verbindungen mit gegenüber Isocyanatgruppen reaktionsfähigen Gruppen eines maximalen Molekulargewichts von 300 und insbesondere von 60-300.

Geeignete Reaktionspartner für die s-Triazineinheiten aufweisenden Polyisocyanate sind insbesondere:

a) Mindestens 2 alkoholische Hydroxylgruppen aufweisende Verbindungen der o.g. Molekulargewichtsbereiche, die keine primären oder sekundären Aminogruppen aufweisen, z.B. einfache Alkanpolyole wie Ethylenglykol, 1,2- oder 1,3-Dihydroxypropan, 1,4-, 1,3- oder 2,3-Dihydroxybutan, 1,6-Dihydroxyhexan, 1,8-Dihydroxyoctan, Neopentylglykol, 1,4-Bis-hydroxy-methyl-cyclohexan, Glycerin, Trimethylolpropan, Hexantriol-(1,2,6), Pentaerythrit, Sorbit, niedermolekulare Ethergruppen aufweisende Polyole wie Diethylenglykol, Dipropylenglykol, Triethylenglykol, Tripropylenglykol, die entsprechenden Polyalkylenglykole mit einem Molekulargewicht bis 300.

b) Hydroxyalkylamine, d.h. Verbindungen, die neben mindestens einer Hydroxylgruppe mindestens eine primäre oder sekundäre Aminogruppe aufweisen, wie z.B. Ethanolamin, Diethanolamin, Isopropanolamin, Diisopropanolamin, N-Hydroxyethyl-ethylendiamin oder N-Hydroxyethyl-hexylendiamin.

c) Mindestens 2 primäre und/oder sekundäre Aminogruppen aufweisende Polyamine der o.g. Molekulargewichtsbereiche wie z.B. Hydrazin, Ethylendiamin, 1,6-Diaminohexan, 2,4- und/oder, 2,6-Diaminotoluol, 2,4'- oder 4,4'- Diaminodiphenylmethan, 2,4- oder 2,6-Hexahydrotoluylendiamin, perhydriertes 2,4'- oder 4,4'-Diaminodiphenylmethan, p-Xylylendiamin, cycloaliphatische Triamine, beispielsweise gemäß DE-OS 2 614 244,

Bei dem erfindungsgemäßen Verfahren können selbstverständliche beliebige Gemische der unter a) bis c) beispielhaft genannten Verbindungen eingesetzt werden.

Bei der Durchführung des erfindungsgemäßen Verfahrens wird durch ein geeignetes Äquivalentverhältnis zwischen Isocyanatgruppen und gegenüber Isocyanatgruppen reaktionsfähigen Gruppen dafür Sorge getragen, daß keine Polyurethane, d.h. hochmolekulare Verbindungen, sondern lediglich vergleichsweise niedermolekulare Isocyanatgruppen oder gegenüber Isocyanatgruppen reaktionsfähige Gruppen aufweisende Verbindungen entstehen. Dies bedeutet, daß beim erfindungsgemäßen Verfahren die Reaktionspartner in einem Äquivalentverhältnis (i) von Isocyanatgruppen zu gegenüber Isocyanatgruppen reaktionsfähigen Gruppen von mindestens 1,5:1, vorzugsweise 2 n:1 bis 4 n:1 oder (ii) einem Äquivalentverhältnis zwischen gegenüber Isocyanatgruppen reaktionsfähigen Gruppen und Isocyanatgruppen von mindestens 1,5:1, vorzugsweise 0,8 n:1 bis 1,2 n:1 zum Einsatz gelangen, wobei n für die Funktionalität der Reaktionspartner mit gegenüber Isocyanatgruppen reaktionsfähigen Gruppen steht. Die Art und Mengenverhältnisse der Reaktionspartner werden im übrigen so gewählt, daß Verfahrensprodukte mit einem maximalen Molekulargewicht von 2000, entstehen. Das Molekulargewicht der Ausgangsmaterialien und der Verfahrensprodukte kann aus der Funktionalität und dem Gehalt an funktionellen Gruppen errechnet werden.

Die Durchführung des erfindungsgemäßen Verfahrens kann beispielsweise dergestalt erfolgen, daß man eine Mischung aus einem Polyisocyanat der o.g. Art und mindestens einer Verbindung mit gegenüber Isocyanatgruppen reaktionsfähigen Gruppen der o.g. Art während eines Zeitraumes von 0,25-25, vorzugsweise 0,5-15 Stunden unter Rühren zur Reaktion bringt. Das erfindungsgemäße Verfahren wird im Falle der Verwendung von primäre oder sekundäre Aminogruppen aufweisenden Reaktionspartnern für die s-Triazineinheiten aufweisenden Polyisocyanate im allgemeinen im Temperaturbereich zwischen 0 und 100°C, vorzugsweise zwischen 20 und 80°C, bei der Verwendung von Hydroxylgruppen aufweisenden Reaktionspartnern im Temperaturbereich zwischen 40 und 190°C, vorzugsweise zwischen 60 und 160°C, durchgeführt. Die Umsetzung kann in Substanz oder in einem gegenüber Isocyanatgruppen inerten Lösungsmittel wie z.B. Dioxan, Tetrahydrofuran, Benzol, Toluol, Chlorbenzol, Dichlorbenzol, Nitrobenzol, Xylol, oder chlorierten aliphatischen Kohlenwasserstoffen durchgeführt werden.

Es ist grundsätzlich jedoch auch möglich, die erfindungsgemäße Umsetzung in Gegenwart eines flüssigen Reaktionsmediums durchzuführen, welches gegenüber Isocyanatgruppen reaktionsfähige Gruppen aufweist, falls die erfindungswesentlichen Reaktionspartner für die s-Triazineinheiten aufweisenden Polyisocyanate gegenüber Isocyanatgruppen eine höhere Reaktionsbereitschaft aufweisen als die gegenüber Isocyanatgruppen reaktionsfähigen Gruppen des Reaktionsmediums. So ist es beispielsweise möglich, als Reaktionsmedium ein Polyesterpolyol oder Polyetherpolyol des Molekulargewichtsbereichs 1000 bis 10 000, vorzugsweise 1000 bis 4000 einzusetzen, und die erfindungsgemäße Umsetzung zwischen s-Triazineinheiten aufweisenden Polyisocyanaten und primäre oder sekundäre Aminogruppen aufweisenden Ausgangsmaterialien der beispielhaft genannten Art durchzuführen. Im Falle der Verwendung von höhermolekularen Polyether- oder Polyesterpolyolen entstehen hierbei in situ Dispersionen der erfindungsgemäßen Verfahrensprodukte in den genannten Polyhydroxylverbindungen.

Im Falle der Verwendung von Ausgangsmaterialien mit primären oder sekundären Aminogruppen ist auch die Verwendung von weit weniger reaktiven niedermolekularen Alkoholen, beispielsweise von Ethanol als Reaktionsmedium möglich, da die Additionsreaktion selektiv zwischen den Aminogruppen und Isocyanatgruppen abläuft.

Beim erfindungsgemäßen Verfahren werden oft die an sich bekannten Beschleuniger für die Isocyanat-Additionsreaktion mitverwendet. Hierzu gehören beispielsweise Amidine wie Diazabicycloundecen, N-Methyl-2-methyl-tetrahydropyrimidin, tert.-Amine wie Triethylendiamin oder Metallkatalysatoren wie Zinn-(II)-octoat, Dibutylzinn-laurat oder Bleidioctoat.

Beim erfindungsgemäßen Verfahren entstehen als Verfahrensprodukte Verbindungen, die im allgemeinen einen Gehalt an s-Triazineinheiten (Molekulargewicht = 78) von ca. 0,1 bis 12, vorzugsweise 4 bis 10 Gew.-% und einen Gehalt an Isocyanatgruppen oder an gegenüber Isocyanatgruppen reaktionsfähigen Gruppen von 1,25 bis 900, vorzugsweise 100 bis 700 Millimol pro 100 g aufweisen.

Je nach Art und Mengenverhältnis der eingesetzten Ausgangsmaterialien entstehen beim erfindungsgemäßen Verfahren einbaufähige Füllstoffe eines breiten Schmelzpunktsbereichs. Die erfindungsgemäßen Verfahrensprodukte unterscheiden sich von den s-Triazineinheiten aufweisenden Ausgangspolyisocyanaten jedoch durch einen verminderten Schmelzpunkt und/oder eine verbesserte Verträglichkeit mit den Ausgangsmaterialien der Polyurethanchemie. Sie eignen sich insbesondere als einbaufähige Füllstoffe bei der Herstellung von Polyurethankunststoffen nach dem Isocyanat-Polyadditionsverfahren, wobei sie, je nach dem, ob es sich um Verbindungen mit Isocyanatgruppen oder mit Verbindungen mit gegenüber Isocyanatgruppen reaktionsfähigen Gruppen handelt, der Polyisocyanat-Komponente bzw. der Komponente mit gegenüber Isocyanatgruppen reaktionsfähigen Gruppen einverleibt werden können. Die erfindungsgemäßen Verfahrensprodukte eignen sich wegen ihres reduzierten Schmelzpunktes auch weit besser als die ihnen zugrundeliegenden s-Triazineinheiten aufweisenden Polyisocyanate gemäß europäischer Patentschrift 893 als Bindemittelkomponente für Zweikomponenten-Polyurethan-Pulverlacke. Die mit den erfindungsgemäßen Verfahrensprodukten hergestellten Polyurethankunststoffe zeichnen sich insbesondere durch verbesserte mechanische Eigenschaften und ein verbessertes Brandverhalten aus.

Die in den nachfolgenden Beispielen gemachten Prozentangaben beziehen sich auf Gewichtsprozente.

**Beispiel 1**

100 g N,N',N''-Tris-(3-isocyanato-4-methyl-phenyl-amino-carbonyl)-melamin (0,154 Mol) werden in 200 ml Ethanol bei Raumtemperatur suspendiert und mit 28,3 g Ethanolamin (0,471 Mol) versetzt. Anschließend wird das Reaktionsgemisch 2 Stunden bei 60° C gerührt. Der Feststoff wird abgesaugt und mit 100 ml Ethanol gewaschen.

Es resultiert ein bei 224 bis 226° C schmelzendes Umsetzungsprodukt mit einem Gehalt an Triazineinheiten von 9.4 % und einem Hydroxylgehalt von 6.5 %.

**Beispiel 2**

100 g des Triisocyanats aus Beispiel 1 (0,154 Mol) werden bei Raumtemperatur in 200 ml Ethanol suspendiert. Anschließend werden 48 g N-Hydroxyethyl-ethylendiamin (0,463 Mol) unter Rühren während eines Zeitraums von 20 Minuten bei Raumtemperatur zugesetzt. Dann wird noch 1 Stunde gerührt und der resultierende Feststoff abgesaugt und mit Ether gewaschen. Es resultieren 130 g einer bei 216 bis 219° C schmelzenden Substanz mit einem Gehalt an Triazineinheiten von 8,1 % und einem Gehalt an Hydroxylgruppen und aminischen Aminogruppen von insgesamt 10 %.

**Beispiel 3**

100 g (0,154 Mol) des Triisocyanats aus Beispiel 1 werden in 200 ml Ethanol suspendiert und während 30 Minuten unter Rühren mit 54,6 g N-Hydroxyethyl-propylen-diamin-(1,3) (0,463 Mol) versetzt. Es wird anschließend 90 Minuten bei 62° C gerührt. Der resultierende Feststoff wird abgesaugt und nacheinander mit 50 ml Aceton und 50 ml Ethanol gewaschen. Es resultieren 142 g einer bei 222 bis 223° C schmelzenden Substanz, die einen Gehalt an Triazineinheiten von 7,8 % und einen Gehalt an Hydroxylgruppen und aminischen Aminogruppen von insgesamt 9,6 % aufweist.

**Beispiel 4**

64,8 g des Triisocyanats aus Beispiel 1 (0,1 Mol) und 100 g Triethylenglykol (0,67 Mol) werden während 90 Minuten bei 140° C gerührt. Der Reaktionsansatz wird anschließend mit Aceton aufgenommen und das Reaktionsprodukt abgesaugt. Anschließend wird mit 50 ml Aceton und schließlich mit 50 ml Ether gewaschen. Es resultiert eine bei 280 bis 300° C schmelzende Substanz mit einem Gehalt an Triazineinheiten von 7,1 und einem Hydroxylgehalt von 4,6 %.

**Beispiel 5**

50 g des Triisocyanats gemäß Beispiel 1 (0,077 Mol) werden in 100 ml Toluol suspendiert. Anschließend werden bei Raumtemperatur 2,31 g Ethylendiamin (0,0385 Mol) zugesetzt. Die Mischung wird anschließend 2 Stunden bei 60° C gerührt. Nach Absaugen und Waschen mit Toluol resultieren 52 g einer bei 288 bis 291° C schmelzenden Substanz mit einem Gehalt an Triazineinheiten von 11,5 % und an NCO-Gruppen von 12,4 %.

**Beispiel 6**

40 g Diethanolamin.(0,38 Mol) werden in 100 ml Dioxan gelöst. Unter Rühren werden bei 50° C portionsweise 83,0 g (0,128 Mol) des Triisocyanats aus Beispiel 1 während eines Zeitraumes von 5 Minuten zugegeben. Es wird 30 Minuten bei 50° C gerührt. Der resultierende Feststoff wird abgesaugt und mit Ether gewaschen. Es resultieren 120 g einer bei 223° C schmelzenden Substanz mit einem Gehalt an Triazineinheiten von 8,1 % und einem Hydroxylgehalt von 10,6 %.

**Beispiele 7 bis 11**

In diesen Beispielen wird das Triisocyanat gemäß Beispiel 1 mit verschiedenen Aminoalkoholen bzw. Diaminoalkanen in einem Polyether A der OH-Zahl 42, hergestellt durch Propoxylierung eines Gemisches aus Trimethylolpropan und Propylenglykol (Molverhältnis = 3:1) und anschließende Ethoxylierung des

Propoxylierungsproduktes, umgesetzt. Hierbei wird stets so verfahren, daß die aminische Reaktionskomponente in Polyether gelöst und mit den in der Tabelle 1 aufgeführten Mengen des Triisocyanats versetzt werden. Die Umsetzung erfolgt jeweils während 30 Minuten unter Rühren bei 50°C. Zur Schmelzpunktbestimmung wurde ein geringer Teil des stets als Dispersion in Polyether anfallenden Feststoffs durch Filtration isoliert und mit Diethylether gewaschen.

**Tabelle 1**

(Mengenangaben in g):

| | Bsp. 7 | Bsp. 8 | Bsp. 9 | Bsp. 10 | Bsp. 11 |
|---|---|---|---|---|---|
| Polyether A | 500 | 500 | 500 | 500 | 500 |
| Diethanolamin | 20 | — | — | — | — |
| Diisopropanol-amin | — | 25,3 | 50,6 | 17,3 | 11,9 |
| Ethylendiamin | — | — | — | 1,8 | 3 |
| Triisocyanat | 41,8 | 41,8 | 83,6 | 41,8 | 41,8 |
| Feststoffge-halt % | 11 | 11,8 | 21,2 | 10,9 | 10,2 |
| Schmp. des Feststoffes | 226 | 350 | 350—50 | 340 | 340 |

**Beispiele 12 bis 17**

In diesen Beispielen wird die Verwendung deringen Beispielen 7 bis 11 hergestellten Suspensionen zur Herstellung harter Polyurethanintegralschaumstoffe gezeigt (Beispiele 12 bis 16). Beispiel 17 ist ein Vergleichsbeispiel ohne Mitverwendung von erfindungsgemäßen Verfahrensprodukten. Die Ergebnisse sind in nachstehender Tabelle 2 zusammengefaßt.

**Tabelle 2**

| | Bsp. 12 | Bsp. 13 | Bsp. 14 | Bsp. 15 | Bsp. 16 | Bsp. 17 |
|---|---|---|---|---|---|---|
| Suspension aus Beispiel 7 | 40 | — | — | — | — | — |
| Suspension aus Beispiel 8 | — | 40 | — | — | — | — |
| Suspension aus Beispiel 9 | — | — | 40 | — | — | — |
| Suspension aus Beispiel 10 | — | — | — | 40 | — | — |
| Suspension aus Beispiel 11 | — | — | — | — | 40 | — |
| Polyether B[1] | 60 | 60 | 60 | 60 | 60 | 60 |
| Polyether A | — | — | — | — | — | 40 |
| OS 50[2] | 1 | 1 | 1 | 1 | 1 | 1 |
| E[3] | 3 | 3 | 3 | 3 | 3 | 3 |
| K[4] | 3 | 3 | 3 | 3 | 3 | 3 |
| TMG[5] | 0,5 | 0,5 | 0,5 | 0,5 | 0,5 | 0,5 |
| $H_3PO_4$ (85%) | 0,2 | 0,2 | 0,2 | 0,2 | 0,2 | 0,2 |
| Trichlorfluormethan | 10 | 10 | 10 | 10 | 10 | 10 |
| Komponente B[6] | 146,5 | 146,5 | 149,2 | 145,3 | 144,5 | 143 |

1) Polyether B = Polyether der OH-Zahl 860, der durch Anlagerung von Propylenoxid an Trimethylolpropan erhalten wurde.
2) OS 50 = Stabilisator der Fa. Goldschmidt, Essen auf Polysiloxan-Polyalkylenoxid-Blockcopolymer-Basis.
3) E = handelsüblicher Emulgator vom Amidamin-Typ.
4) K = Aminkatalysator
5) TMG = Tetramethylguanidin
6) Polyisocyanat, das durch Phosgenierung von Anilin-Formaldehyd-Kondensaten erhalten wurde und eine Viskosität von 130 mPas bei 25° C und einem NCO-Wert von 31 Gew.-% aufweist.

Die in Tabelle 2 erstgenannten Verbindungen werden jeweils zur Komponente A gemischt und dann mit einem Schnellrührer zur Luftbeladung ca. 30 Sekunden gerührt. Dann wird die angegebene Menge der Komponente B zugegeben, die Mischung wird ca. 10 bis 15 Sekunden intensiv vermischt und dann in eine offene Papierform (Maße 250x120 x120 mm) eingetragen. Bei der nun einsetzenden Schaumbildung werden die Startzeit (Zeit zwischen Beginn der Vermischung von Komponente A und B und Beginn der Schaumbildung) und Fadenziehzeit (Zeit zwischen Beginn der Vermischung von Komponente A und B und dem Zeitpunkt, an dem ein im Sekundenabstand in den aufsteigenden Schaum eingetauchter Holzstab (Ø ca. 2 mm) beim Herausnehmen aus dem Schaum Fäden zieht, ermittelt.

Zum Herstellen einer Prüfplatte wird das Gemisch aus Komponenten A und B direkt nach dem Verrühren in ein senkrecht stehendes, auf ca. 60° C thermostatisiertes Aluminium-Formwerkzeug gegossen, welches eine platte der Größe 200x200x10 $mm^3$ liefert. Nach einer Standzeit von 10 Minuten wird die Platte aus dem Formwerkzeug entnommen und 2 Tage bei Raumtemperatur und normaler Luftfeuchtigkeit gelagert. Dann wird die Oberflächenhärte gemessen und ein 200x100x10 $mm^3$ großer Teil der Probeplatte einem Wärmestandtest unterworfen, wobei die Platte mit dem 100 mm langen Kanten auf einem U-förmigen Holzgestell gelagert wird (ca. 5 mm Auflage an den Kanten) und in der Mitte mit einem 1 kg Gewichtsstück bei 110°C während 1 Stunde

belastet wird. Nach dem Test wird die Durchbiegung der Platte in Plattenmitte in mm ermittelt und nach der Formel Durchbiegung $_{(RG)}$

$$\cdot \frac{RG}{600}$$

auf das Raumgewicht 600 normiert.
Folgende Daten wurden erhalten:

| | Bsp. 12 | Bsp. 13 | Bsp. 14 | Bsp. 15 | Bsp. 16 | Bsp. 17 |
|---|---|---|---|---|---|---|
| Startzeit (sek) | 24 | 22 | 22 | 20 | 21 | 24 |
| Fadenziehzeit (sek) | 38 | 33 | 35 | 31 | 32 | 40 |

**Probeplatte**

| | | | | | | |
|---|---|---|---|---|---|---|
| Beispiel | 12 | 13 | 14 | 15 | 16 | 17 |
| RG | 510 | 530 | 550 | 510 | 580 | 580 |
| Shore D | 64 | 66 | 67 | 64 | 69 | 71 |
| Durchbiegung (mm) | 2,5 | 1,5 | 1,0 | 3,0 | 2,1 | 3,5 |
| auf 600 norm. | 2,125 | 1,325 | 0,917 | 2,55 | 2,03 | 3,38 |

**Patentansprüche**

1) Verfahren zur Herstellung von unzersetzt schmelzbaren, als einbaufähige Füllstoffe für Polyurethankunststoffe geeigneten, s-Triazineinheiten und Isocyanatgruppen oder gegenüber Isocyanatgruppen reaktionsfähige Gruppen aufweisenden Verbindungen mit einem maximalen Molekulargewicht von 2000, dadurch gekennzeichnet, daß man Triisocyanate der Formel

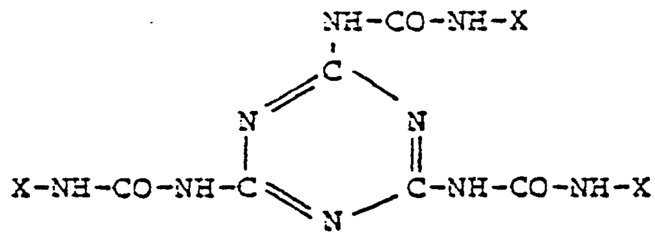

in welcher

X für einen Rest steht, wie er durch Entfernung der höher-reaktiven Isocyanatgruppe aus einem aromatischen Diisocyanat mit Isocyanatgruppen unterschiedlicher Reaktivität im Sinne der Isocyanat-Additionsreaktion erhalten wird,

mit mindestens difunktionellen Verbindungen eines maximalen Molekulargewichts von 300 mit gegenüber Isocyanatgruppen reaktionsfähigen Gruppen unter Einhaltung eines Äquivalentverhältnisses zwischen Isocyanatgruppen und gegenüber Isocyanatgruppen reaktionsfähigen Gruppen von mindestens 1,5:1 oder unter Einhaltung eines Äquivalentverhältnisses zwischen gegenüber Isocyanatgruppen reaktionsfähigen Gruppen und Isocyanatgruppen von mindestens 1,5:1 zur Reaktion bringt.

2) Verfahren gemäß Anspruch 1, dadurch gekennzeichnet, daß man als Verbindungen mit gegenüber Isocyanatgruppen reaktionsfähigen Gruppen solche ausgewählt

aus

a) Polyhydroxylverbindungen, die keine primäre oder sekundäre Aminogruppen aufweisen,

b) primäre und/oder sekundäre Aminogruppen aufweisenden Hydroxyalkylaminen

c) Polyaminen mit mindestens 2 primären und/oder sekundären Aminogruppen und

d) beliebigen Gemischen der unter a) bis c) genannten Verbindungen

verwendet.

3) Verfahren gemäß Ansprüchen 1 und 2, dadurch gekennzeichnet, daß man als Verbindungen der in Anspruch 1 genannten Formel solche verwendet, für welche

X für einen 3-Isocyanato-4-methyl-phenyl- oder einen 4-(2-Isocyanatobenzyl)-phenylrest steht.

4) Verwendung der gemäß Ansprüchen 1 bis 3 erhältlichen Verbindungen mit Isocyanatgruppen oder mit gegenüber Isocyanatgruppen reaktionsfähigen Gruppen als einbaufähige Füllstoffe bei der Herstellung von Polyurethankunststoffen nach dem Isocyanat-Polyadditionsverfahren.

**Claims**

1. Process for the preparation of compounds which contain s-triazine units and isocyanate groups or groups which are reactive towards isocyanate groups, are suitable as fillers which can be incorporated into polyurethane plastics, can be melted without decomposition and have a maximum molecular weight of 2,000, characterised in that triisocyanates of the formula

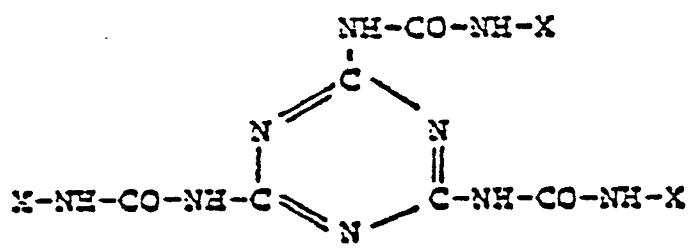

in which

X represents a radical such as is obtained by removing the more highly reactive isocyanate group from an aromatic diisocianate containing isocyanate groups of differing reactivity with respect to the isocyanate addition

reaction, are reacted with at least difunctional compounds having a maximum molecular weight of 300 and containing groups which are reactive towards isocyanate groups, while adhering to an equivalent ratio between isocyanate groups and groups which are reactive towards isocyanate groups of at least 1.5:1 or while adhering to an equivalent ratio between groups which are reactive towards isocyanate groups and isocyanate groups of at least 1.5:1.

2. Process according to Claim 1, characterised in that the compounds used as the compounds containing groups which are reactive towards isocyanate groups are selected from

a) polyhydroxyl compounds which do not contain any primary or secondary amino groups,
b) hydroxyalkylamines containing primary and/or secondary amino groups,
c) polyamines containing at least 2 primary and/or secondary amino groups and
d) any desired mixtures of the compounds mentioned under a) to c).

3. Process according to Claims 1 and 2, characterised in that compounds for which X represents a 3-isocyanato-4-methyl-phenyl or 4-(2-isocyanatobenzyl)-phenyl radical, are used as the compounds of the formula mentioned in Claim 1.

4. Use of the compounds obtainable according to Claims 1 to 3 containing isocyanate groups or groups which are reactive towards isocyanate groups as incorporable fillers in the production of polyurethane plastics by the isocyanate polyaddition process.

**Revendications**

1. Procédé de préparation de composés fusibles sans décomposition, appropriés comme charges incorporables pour les matières synthétiques de polyuréthanes, comportant des motifs s-triazine et des groupes isocyanate ou des groupes réactifs vis-à-vis des groupes isocyanate et ayant un poids moléculaire maximum de 2.000, caractérisé en ce qu'on fait réagir des triisocyanates de formule :

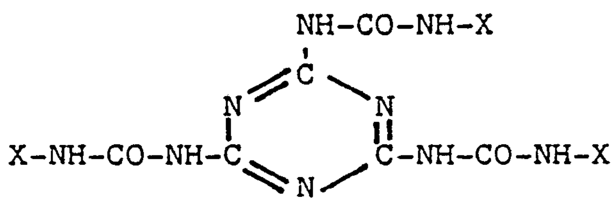

dans laquelle

X représente un radical tel que celui obtenu par élimination du groupe isocyanate réactif supérieur hors d'un diisocyanate aromatique comportant des groupes isocyanate d'une réactivité différente dans le sens de la réaction d'addition d'isocyanate, avec des composés au moins difonctionnels d'un poids moléculaire maximum de 300 comportant des groupes reactifs vis-à-vis des groupes isocyanate, tout en maintenant, entre les groupes isocyanate et les groupes réactifs vis-à-vis des groupes isocyanate, un rapport d'équivalents d'au moins 1,5:1 ou tout en maintenant, entre les groupes réactifs vis-à-vis des groupes isocyanate et les groupes isocyanate , un rapport d'équivalents d'au moins 1,5:1.

2. Procédé selon la revendication 1, caractérisé en ce que, comme composés comportant des groupes réactifs vis-à-vis des groupes isocyanate, on utilise ceux choisis parmi :

a) les composés polyhydroxy ne comportant aucun groupe amino primaire ou secondaire,
b) les hydroxy-alkylamines comportant des groupes amino primaires et/ou secondaires,
c) les polyamines comportant au moins deux groupes amino primaires et/ou secondaires, et
d) n'importe quels mélanges des composés mentionnés sub a) à c).

3. Procédé selon les revendications 1 et 2, caractérisé en ce que, comme composés répondant à la formule indiquée dans la revendication 1, on utilise ceux dans lesquels

X représente un radical 3-isocyanato-4-méthyl-phényle ou un radical 4-(2-isocyanatobenzyl)-phényle.

4. Utilisation des composés obtenus selon les revendications 1 à 3 et comportant des groupes isocyanate ou des groupes réactifs vis-à-vis des groupes isocyanate comme charges incorporables lors de la préparation de matières synthétiques de polyuréthanes selon le procédé de poly-addition d'isocyanate.